# EUROPEAN PATENT APPLICATION

(11) **EP 4 480 496 A1**
(43) Date of publication of application: **25.12.2024**
(21) Application number: 23755862.2
(22) Date of filing: 17.02.2023
(51) Int. Cl.: A61K 47/52, A61K 47/60, A61K 47/69, A61K 38/12, A61K 38/20, A61K 38/06, A61K 38/19, A61P 35/00

(54) **ALUMINUM NANOCRYSTALLINE COMPOSITE IMMUNE DRUG AND PREPARATION METHOD THEREFOR AND USE THEREOF**

(30) Priority: 18.02.2022 CN 202210152866
(71) Applicant: Guangzhou Realbenefitspot Pharmaceutical Co., Ltd., Guangzhou, Guangdong 510663 (CN)
(72) Inventor: WANG, Yaling, Guangzhou, Guangdong 510000 (CN); CHEN, Chunying, Guangzhou, Guangdong 510000 (CN); ZHAO, Yuliang, Guangzhou, Guangdong 510000 (CN)
(74) Representative: J A Kemp LLP
(86) International application number: PCT/CN2023/076660
(87) International publication number: WO 2023/155861

(57) **Abstract**

The present invention relates to the technical field of biomedicines and vaccines, and in particular to an aluminum nanocrystalline composite immune drug and a preparation method therefor and use thereof. The aluminum nanocrystalline is used as a carrier, the surface of the aluminum nanocrystalline is covered with polyethylene glycol, and functional polypeptide sequences and cytokine molecules are linked by a polyethylene glycol end group to form a virus-like particle immune drug. According to the drug, the cytokine stability and the tumor tissue retention time are improved, and the auxiliary anti-tumor effect of the cytokines is effectively improved.

## Description

This application claims the priority of Chinese patent application No. 202210152866.9, filed with the China Patent Office on February 18, 2022, entitled "ALUMINUM NANOCRYSTALLINE COMPOUND IMMUNE DRUG AND PREPARATION METHOD THEREFOR AND USE THEREOF", which is incorporated herein by reference in its entirety.

### Technical Field

The present invention relates to the technical field of biomedicines and vaccines, and in particular to an aluminum nanocrystalline composite immune drug and a preparation method therefor and use thereof.

### Background

To date, although great progress has been made in theoretical research and related technologies in clinical medicine, tumors (cancers) are still diseases that are difficult to cure and have a high mortality ratio. Clinically, there are three traditional therapeutic regimens for tumors: surgery, chemistry, and radiation. Surgical therapy involves direct removal of an affected site through surgery. It is more effective for treating early-stage tumors or benign tumors with low spread ability. However, for cancer patients with metastasis, the efficacy and prognosis of quality of life are poor. Due to its lack of specificity, chemotherapy also causes significant damage to a patient's own immune system and stem cells while treating tumors. In recent years, with the continuous in-depth research on the growth and development process of tumors, immunotherapy, an innovative therapeutic regimen, has been proposed, which has greatly promoted the development of cancer treatment. In 2013, it was ranked first among the top ten scientific breakthroughs of the year by Science magazine. It is one of the most important research directions in clinical treatment of cancers in recent years.

Immunotherapy is a general term for a large class of treatment approaches that treat cancers by activating the human immune system. The human immune system has always acted as a "police officer" to drive away e.g., foreign invaders from the body. Tumor immunotherapy works in three ways: (1) designing monoclonal antibodies to enhance immune responses to destroy cancer cells; (2) using immune checkpoint inhibitors to help the immune system recognize and attack cancer cells; and (3) synthesizing cancer vaccines to stimulate immune responses to treat and prevent cancers. Unlike other cancer treatment approaches, immunotherapy mainly activates the immune system to give the human body the ability to attack tumor cells. Due to the memory function of the human immune system, the process may continue for a long time after the initial treatment. In addition, the immune system has the ability to distinguish cancer cells from normal cells and can selectively attack cancer cells and avoid damaging normal cells by activating immune responses.

Cytokines are one of the major players in regulating immune responses, and cytokine-based approaches provide an alternative strategy for cancer immunotherapy. Over the past few decades, cytokines and cytokine receptors have been extensively studied as targets for cancer therapy. Cytokines are a class of small-molecule proteins with a wide range of biological activities that are expressed and secreted by immune cells (such as monocytes, macrophages, T cells, B cells, NK cells, etc.) and certain specific non-immune cells (endothelial cells, epidermal cells, fibroblasts, etc.) upon stimulation. They can regulate innate and adaptive immunity, hematopoiesis, cell growth, APSC pluripotent cells, and repair of damaged tissues by binding to respective receptors. Cytokines can be divided into interleukins, interferons, some members of the tumor necrosis factor superfamily, colony stimulating factors, chemokines, growth factors, etc. Cytokines are key mediators of cellular communication in the tumor microenvironment. Dysregulated cytokine production by malignant cells, immune cells, and stromal cells is involved in all stages of tumor initiation and development. Certain cytokines are closely associated with tumor development, progression, and metastasis, and aberrant production of inflammatory cytokines is a common downstream consequence of oncogenic changes in non-malignant cells. Therefore, utilizing the immunostimulatory effects of cytokines and neutralizing their effects when cytokines are dysregulated can effectively achieve the treatment of tumor tissues. However, systemic administration of cytokines lacks targeting, has certain systemic toxicity, and has potential adverse effects on embryonic tissues. Therefore, local administration of cytokine drugs is a relatively effective and safe method.

Therefore, it will be of great practical value to develop a composite immune drug based on an aluminum nanocrystalline to solve the problems of cytokine stability in the body and local accumulation time, increase the time of cytokine action with cells in tumor tissues, and play the role of immune regulation fully.

### Summary of the Invention

In view of this, the present invention provides an aluminum nanocrystalline composite immune drug and a preparation method therefor and use thereof, as well as functional polypeptide sequences and cytokines linked to the surface of the aluminum nanocrystalline in the composite immune drug based on the aluminum nanocrystalline. The aluminum nanocrystalline provided by the present invention can effectively bind to functional polypeptide sequences and cytokines through polyethylene glycol molecules to construct a composite immune drug, effectively improve the binding ability of the composite immune drug to tumor cells at tumor sites and increase the accumulation time of the composite immune drug at tumor sites by functional polypeptide sequences; while effectively increasing the stability of cytokines and the time window for them to exert immunoregulatory ability at tumor sites, so as to improve the immune responses of an organism to tumor tissues effectively, and inhibit the growth of tumor tissues.

In order to achieve the above-mentioned object of the invention, the present invention provides the following technical solutions:
The present invention provides an aluminum nanocrystalline composite immune drug, which is constructed by binding a functional polypeptide sequence and a cytokine molecule based on aluminum nanocrystals by means of polyethylene glycol molecule.

Further, in the aluminum nanocrystalline composite immune drug, the functional polypeptide sequence comprises a polypeptide sequence which can specifically bind to a protein highly expressed on the surface of tumor cells.

Further, in the aluminum nanocrystalline composite immune drug, the functional polypeptide sequence is one or both of a circular RGD sequence and a TAT sequence that can assist an immune drug to enter cells.

Further, in the aluminum nanocrystalline composite immune drug, the cytokine molecule is selected from one or more of interleukin factors including IL-2, IL-7, IL-12, IL-15, IL-18, IL-21, gamma interferon IFN-γ, or tumor necrosis factor TNFα.

Further, the construction is based on formation of a covalent amide bond between the amino group of M-PEG-NH₂ on the surface of the aluminum nanocrystals and the carboxyl group on the cytokine molecule.

Further, the aluminum nanocrystals are prepared by the following steps:
stirring and fully mixing an aluminum salt solution and a polyethylene glycol solution, and adding an aqueous NaOH solution to adjust the pH value, and then allowing them to stand to obtain a precipitate, which is then centrifuged, washed and purified to obtain polyethylene glycol-modified aluminum nanocrystals.

Further, the solute of the aluminum salt solution is one or more of aluminum chloride, aluminum nitrate, aluminum sulfate, and aluminum acetate; the solvent used for the aluminum salt solution is pure water or a sodium acetate solution with a concentration of 0.01 mol/L.

Further, the polyethylene glycol is a mixed reagent of polyethylene glycol modified with phosphoserine (PS-PEG) and polyethylene glycol with maleimide group and terminal carboxylic acid (M-PEG-NH₂).

Further, the aqueous NaOH solution is added to adjust the pH to 5.0-8.0.

The present invention also provides a method for preparing the aluminum nanocrystalline composite immune drug, comprising the following steps:
(1) adding aluminum nanocrystals and a functional polypeptide sequence into a solvent, stirring for the reaction, and centrifuging, washing and purifying, then resuspending into ultrapure water to obtain a functional polypeptide sequence-modified aluminum nanocrystalline solution; and
(2) adding a cytokine molecule into an MES buffer, and adding EDC/NHS to activate the carboxyl groups on the cytokine molecule, and then adding the aluminum nanocrystalline solution to form a covalent amide bond between the amino group of M-PEG-_{NH2}and the carboxyl group on the cytokine molecule, thereby obtaining a cytokine molecule-modified aluminum nanocrystalline composite immune drug.

Further, the solvent is 0.1 M phosphate buffer or 0.1 M ammonium acetate buffer.

Further, the functional polypeptide sequence comprises a polypeptide sequence which can specifically bind to a protein highly expressed on the surface of tumor cells.

Further, the functional polypeptide sequence is one or both of a circular RGD sequence and a TAT sequence that can assist an immune drug to enter cells.

Further, the cytokine molecule is selected from one or more of interleukin factors including IL-2, IL-7, IL-12, IL-15, IL-18, IL-21, gamma interferon IFN-γ, and tumor necrosis factor TNFα.

The present invention also provides use of the aluminum nanocrystalline composite immune drug described above or the aluminum nanocrystalline composite immune drug prepared by the preparation method in immunotherapy in tumor models.

The use is intravenous injection, mucosal administration, subcutaneous injection, intradermal injection, peritumoral injection, or intratumoral injection.

The method for immunotherapy of tumors comprises administering the aluminum nanocrystalline composite immune drug or the aluminum nanocrystalline composite immune drug prepared by the preparation method.

Further, the mode of the administration comprises: intravenous injection, mucosal administration, subcutaneous injection, intradermal injection, peritumoral injection, or intratumoral injection.

The technical effects of the present invention are that:
The aluminum nanocrystalline prepared with the aid of polyethylene glycol provided by the present invention can effectively bind to functional polypeptide sequences and cytokines, effectively increase the stability of cytokines and the time window for them to exert immunoregulatory ability at tumor sites, so as to improve the immune responses of an organism to tumor tissues, and inhibit the growth of tumor tissues.

### Description of the Drawings

Fig. 1 shows the analysis of the effect of the aluminum nanocrystalline composite immune drug constructed in Examples 6 and 7 of the present invention on inhibiting tumor growth;
Fig. 2 shows the analysis of the effect of the aluminum nanocrystalline composite immune drug constructed in Examples 8-13 of the present invention on inhibiting tumor growth;
Fig. 3 shows the analysis of cytokine accumulation and stability of the aluminum nanocrystalline composite immune drug constructed in Examples 6 and 7 of the present invention in a mouse tumor model;
Fig. 4 shows the regulation of the aluminum nanocrystalline composite immune drug constructed in Examples 6 and 7 of the present invention on the proliferation of CD8 T cells in tumor tissues in a mouse tumor model;
Fig. 5 shows an analysis of the effect of the aluminum nanocrystalline composite immune drug constructed in Examples 6 and 7 of the present invention on inhibiting distal tumor tissue growth; and
Fig. 6 shows a schematic diagram of the construction process of the aluminum nanocrystalline composite immune drug of the present invention.

### Detailed Description

The present invention discloses an aluminum nanocrystalline composite immune drug and a preparation method therefor, as well as functional polypeptide sequences and cytokines linked to the surface of the aluminum nanocrystalline in the composite immune drug based on the aluminum nanocrystalline. Those skilled in the art can refer to the contents of this article and appropriately improve the process parameters to achieve the desired effect. It should be particularly noted that all similar substitutions and modifications are obvious to those skilled in the art, and they are deemed to be included in the present invention. The method and use of the present invention have been described through preferred examples. Related persons can obviously modify or appropriately change and combine the methods and use described herein without departing from the content, spirit and scope of the present invention to implement and apply the technology of the present invention.

The raw materials and reagents used in Examples 1 to 19 provided by the present invention can all be purchased from the market.

The present invention will be further described below in conjunction with examples:

### Example 1 Preparation of aluminum nanocrystalline (Al-PEG-1)

AlCl₃·6H₂O was dissolved in 0.01 mol/L NaAc to prepare an aluminum salt solution with an aluminum ion content of 0.05 mol/L; a mixed solution of PS-PEG with a total molecular concentration of 0.05 mol/L and M-PEG-NH₂with a molar ratio of 1:1 was added to the obtained aluminum salt solution, wherein the molar ratio between Al³⁺ and phosphoserine was 1:2; a aqueous NaOH solution was added to adjust the pH value to 7.6, and after the reaction was completed, the mixture was allowed to stand for 7 hours, centrifuged and washed, and finally sterilized by high-pressure steam or through a filter membrane to obtain an aluminum nanocrystalline, named as Al-PEG-1.

### Example 2 Preparation of aluminum nanocrystalline (Al-PEG-2)

Al(NO₃)₃ was dissolved in pure water to prepare an aluminum salt solution with an aluminum ion content of 0.05 mol/L; a mixed solution of PS-PEG with a total molecular concentration of 0.05 mol/L and M-PEG-NH₂with a molar ratio of 1:2 was added to the obtained aluminum salt solution, wherein the molar ratio between Al³⁺ and phosphoserine was 1: 1; a aqueous NaOH solution was added to adjust the pH value to 5.0, and after the reaction was completed, the mixture was allowed to stand for 10 hours, centrifuged and washed, and finally sterilized by high-pressure steam or through a filter membrane to obtain an aluminum nanocrystalline, named as Al-PEG-2.

### Example 3 Preparation of aluminum nanocrystalline (Al-PEG-3)

In this example, an aluminum nanocrystalline (Al-PEG-3) was prepared. The preparation method was substantially the same as that in Example 1, except that the aluminum salt used was aluminum acetate, the molar ratio of PS-PEG to M-PEG-NH₂ was 2: 1, the aqueous NaOH solution was added to adjust the pH value to 8.0, and after the reaction was completed, the standing time was 3 hours.

### Example 4 Preparation of aluminum nanocrystalline (Al-PEG-4)

In this example, an aluminum nanocrystalline (Al-PEG-4) was prepared. The preparation method was substantially the same as that in Example 1, except that the aluminum salt used was aluminum sulfate, the aqueous NaOH solution was added to adjust the pH value to 6.5, and after the reaction was completed, the standing time was 8 hours.

### Example 5

The aluminum nanocrystals or aluminum nanocrystalline composite immune drugs obtained in Examples 1-4 were tested for particle size.

At 25°C, the concentration of the aluminum nanocrystalline was diluted to 10 µg/mL, and the particle size of the aluminum nanocrystalline was tested using a nanoparticle size analyzer.

Wherein, the physical and chemical properties of the aluminum nanocrystals prepared in Examples 1, 2, 3, and 4 are shown in Table 1 below.

**Table 1**

| Detection items | Example 1 Al-PEG-1 | Example 2 Al-PEG-2 | Example 3 Al-PEG-3 | Example 4 Al-PEG-4 |
|---|---|---|---|---|
| Average hydration particle size | 33.7 nm | 38.2 nm | 29.4 nm | 30.1 nm |

As can be seen from Table 1, the preparation examples 1, 2, 3, and 4 of the present invention all obtain nano-sized aluminum nanocrystals with similar particle sizes. Since the main reaction substrates used are similar, the properties of the obtained aluminum nanocrystals are similar.

### Example 6 Preparation of aluminum nanocrystalline composite immune drug Al-RGD-IL12

(1) The aluminum nanocrystalline prepared in Example 1 and the polypeptide sequence c (RGDfC) that can specifically bind to the functional protein integrin highly expressed on the surface of tumor cells were added into 0.1 M phosphate buffer or 0.1 M ammonium acetate buffer, wherein the ratio of the polypeptide sequence to the maleimide contained in the PEG molecule on the surface of the aluminum nanocrystalline was 1.2:1, and the reaction was stirred for 24 hours, centrifuged, washed and purified to obtain an aluminum nanocrystalline modified with the RGD peptide sequence; and
(2) the cytokine IL-12 molecule was added into 50 mM MES buffer, and EDC/NHS was added to activate the carboxyl groups on the molecule, and then the aluminum nanocrystalline solution obtained in step (1) was added, wherein the ratio of M-PEG-NH₂ on the surface of the aluminum nanocrystalline to the cytokine molecule was 1.5:1, thereby obtaining an aluminum nanocrystalline composite immune drug further modified with the cytokine molecule, which was named as Al-RGD-IL12.

### Example 7 Preparation of aluminum nanocrystalline composite immune drug Al-TAT-TNFα

(1) The aluminum nanocrystalline prepared in Example 1 and the membrane-penetrating peptide sequence TAT which could effectively carry nanoparticles into cells were added into 0.1 M phosphate buffer or 0.1 M ammonium acetate buffer, wherein the ratio of the polypeptide sequence to the maleimide contained in the PEG molecule on the surface of the aluminum nanocrystalline was 1.5:1, and the reaction was stirred for 24 hours, centrifuged, washed and purified to obtain an aluminum nanocrystalline modified with the TAT peptide sequence; and
(2) the tumor necrosis factor TNFα molecule was added into 50 mM MES buffer, and EDC/NHS was added to activate the carboxyl groups on the molecule, and then the aluminum nanocrystalline solution obtained in step (1) was added, wherein the ratio of M-PEG-NH₂ on the surface of the aluminum nanocrystalline to the cytokine molecule was 1.3:1, thereby obtaining an aluminum nanocrystalline composite immune drug further modified with the cytokine molecule, which was named as Al-TAT-TNFα.

### Example 8 Preparation of aluminum nanocrystalline composite immune drug Al-TAT-IL2

(1) The aluminum nanocrystalline prepared in Example 2 and the membrane-penetrating peptide sequence TAT which could effectively carry nanoparticles into cells were added into 0.1 M phosphate buffer or 0.1 M ammonium acetate buffer, wherein the ratio of the polypeptide sequence to the maleimide contained in the PEG molecule on the surface of the aluminum nanocrystalline was 1.1:1, and the reaction was stirred for 15 hours, centrifuged, washed and purified to obtain an aluminum nanocrystalline modified with the TAT peptide sequence; and
(2) the cytokine IL2 molecule was added into 50 mM MES buffer, and EDC/NHS was added to activate the carboxyl groups on the molecule, and then the aluminum nanocrystalline solution obtained in step (1) was added, wherein the ratio of M-PEG-NH₂ on the surface of the aluminum nanocrystalline to the cytokine molecule was 1.2:1, thereby obtaining an aluminum nanocrystalline composite immune drug further modified with the cytokine molecule, which was named as Al-TAT-II,2.

### Example 9 Preparation of aluminum nanocrystalline composite immune drug Al-RGD-IL15

(1) The aluminum nanocrystalline prepared in Example 4 and c (RGDfC) were added into 0.1 M phosphate buffer or 0.1 M ammonium acetate buffer, wherein the ratio of the polypeptide sequence to the maleimide contained in the PEG molecule on the surface of the aluminum nanocrystalline was 1.5:1, and the reaction was stirred for 24 hours, centrifuged, washed and purified to obtain an aluminum nanocrystalline modified with the RGD peptide sequence; and
(2) the cytokine IL15 molecule was added into 50 mM MES buffer, and EDC/NHS was added to activate the carboxyl groups on the molecule, and then the aluminum nanocrystalline solution obtained in step (1) was added, wherein the ratio of M-PEG-NH₂ on the surface of the aluminum nanocrystalline to the cytokine molecule was 1.3:1, thereby obtaining an aluminum nanocrystalline composite immune drug further modified with the cytokine molecule, which was named as Al-RGD-IL15.

### Example 10 Preparation of aluminum nanocrystalline composite immune drug Al-TAT-IL18

(1) The aluminum nanocrystalline prepared in Example 3 and the membrane-penetrating peptide sequence TAT which could effectively carry nanoparticles into cells were added into 0.1 M phosphate buffer or 0.1 M ammonium acetate buffer, wherein the ratio of the polypeptide sequence to the maleimide contained in the PEG molecule on the surface of the aluminum nanocrystalline was 1.3:1, and the reaction was stirred for 20 hours, centrifuged, washed and purified to obtain an aluminum nanocrystalline modified with the TAT peptide sequence; and
(2) the cytokine IL18 molecule was added into 50 mM MES buffer, and EDC/NHS was added to activate the carboxyl groups on the molecule, and then the aluminum nanocrystalline solution obtained in step (1) was added, wherein the ratio of M-PEG-NH₂ on the surface of the aluminum nanocrystalline to the cytokine molecule was 1.4:1, thereby obtaining an aluminum nanocrystalline composite immune drug further modified with the cytokine molecule, which was named as Al-TAT-IL18.

### Example 11 Preparation of aluminum nanocrystalline composite immune drug Al-RGD-IL21

(1) The aluminum nanocrystalline prepared in Example 2 and c (RGDfC) were added into 0.1 M phosphate buffer or 0.1 M ammonium acetate buffer, wherein the ratio of the polypeptide sequence to the maleimide contained in the PEG molecule on the surface of the aluminum nanocrystalline was 1.5:1, and the reaction was stirred for 24 hours, centrifuged, washed and purified to obtain an aluminum nanocrystalline modified with the RGD peptide sequence; and
(2) the cytokine IL21 molecule was added into 50 mM MES buffer, and EDC/NHS was added to activate the carboxyl groups on the molecule, and then the aluminum nanocrystalline solution obtained in step (1) was added, wherein the ratio of M-PEG-NH₂ on the surface of the aluminum nanocrystalline to the cytokine molecule was 1.3:1, thereby obtaining an aluminum nanocrystalline composite immune drug further modified with the cytokine molecule, which was named as Al-RGD-IL21.

### Example 12 Preparation of aluminum nanocrystalline composite immune drug Al-RT-IFN-γ

(1) The aluminum nanocrystalline prepared in Example 4 and c (RGDfC) with the TAT peptide (the molar ratio of 1:1) were added into 0.1 M phosphate buffer or 0.1 M ammonium acetate buffer, wherein the ratio of the polypeptide sequence to the maleimide contained in the PEG molecule on the surface of the aluminum nanocrystalline was 1.3:1, and the reaction was stirred for 24 hours, centrifuged, washed and purified to obtain an aluminum nanocrystalline modified with the RGD peptide sequence; and
(2) the gamma interferon IFN-γ molecule was added into 50 mM MES buffer, and EDC/NHS was added to activate the carboxyl groups on the molecule, and then the aluminum nanocrystalline solution obtained in step (1) was added, wherein the ratio of M-PEG-NH₂ on the surface of the aluminum nanocrystalline to the cytokine molecule was 1.5:1, thereby obtaining an aluminum nanocrystalline composite immune drug further modified with the cytokine molecule, which was named as Al-RT-IFN-γ.

### Example 13 Preparation of aluminum nanocrystalline composite immune drug Al-RT-IL7

(1) The aluminum nanocrystalline prepared in Example 1 (the product obtained in Example 1 was only used as a representative in subsequent experiments, and did not mean that only the aluminum nanocrystalline obtained in Example 1 could be used for subsequent experiments) and c (RGDfC) with the TAT peptide (the molar ratio of 1.5:1) were added into 0.1 M phosphate buffer or 0.1 M ammonium acetate buffer, wherein the ratio of the polypeptide sequence to the maleimide contained in the PEG molecule on the surface of the aluminum nanocrystalline was 1.5:1, and the reaction was stirred for 24 hours, centrifuged, washed and purified to obtain an aluminum nanocrystalline modified with the RGD peptide sequence; and
(2) the cytokine IL7 molecule was added into 50 mM MES buffer, and EDC/NHS was added to activate the carboxyl groups on the molecule, and then the aluminum nanocrystalline solution obtained in step (1) was added, wherein the ratio of M-PEG-NH₂ on the surface of the aluminum nanocrystalline to the cytokine molecule was 1.3:1, thereby obtaining an aluminum nanocrystalline composite immune drug further modified with the cytokine molecule, which was named as Al-RT-IL7.

### Example 14

After similar hydration particle size detection as in Example 5, the physical and chemical properties of the products obtained in Examples 6 to 13 are shown in Table 2 below. It can be seen from the data in the table that the particle sizes of aluminum nanocrystalline composite immune drugs prepared from different cytokines or interferons all have increased significantly.

**Table 2**

| Detecti on items | Exam ple 6 Al-RGD-IL12 | Exam ple 7 Al-TAT-TNFα | Exam ple 8 Al-TAT-II,2 | Exam ple 9 Al-RGD-IL15 | Exampl e 10 Al-TAT-IL18 | Exampl e 11 Al-RGD-IL21 | Exampl e 12 Al-RT-IFN-γ | Examp le 13 Al-RT-IL7 |
|---|---|---|---|---|---|---|---|---|
| Averag e hydrati on particle size | 71.4 nm | 68.5 nm | 75.3 nm | 68.4 nm | 62.9 nm | 76.1 nm | 61.2 nm | 68.5 nm |

### Example 15

The aluminum nanocrystalline composite immune drugs constructed above (represented by the Al-RGD-IL12 composite immune drug constructed in Example 6 and the Al-TAT-TNFα composite immune drug constructed in Example 7) were used in immunotherapy in tumor models to evaluate tumor growth inhibition.

Under the premise of following the national animal health agreement, BALB/c mice aged 6-8 weeks were selected for posterior subcutaneous tumor implantation. The tumor model was selected from B 16F 10 mouse melanoma highly metastatic cells. When the implanted tumor tissue grew to about 100 mm³, the mice were randomly divided into 5 groups and injected with 50 µL of the following drugs intratumorally: ①, saline for injection (Ctrl); ②, a simple mixture of the commercial aluminum adjuvant Alum and 0.25 µg IL-12 (Alum-IL12); ③, the Al-RGD-IL12 composite immune drug containing 0.25 µg IL-12 constructed in Example 6; ④, a simple mixture of commercial aluminum adjuvant Alum and 0.25 µg TNFα (Alum-TNFα); and (5), the Al-TAT-TNFα composite immune drug containing 0.25 µg IL-12 constructed in Example 7. The changes in the tumor volume of the mice were recorded.

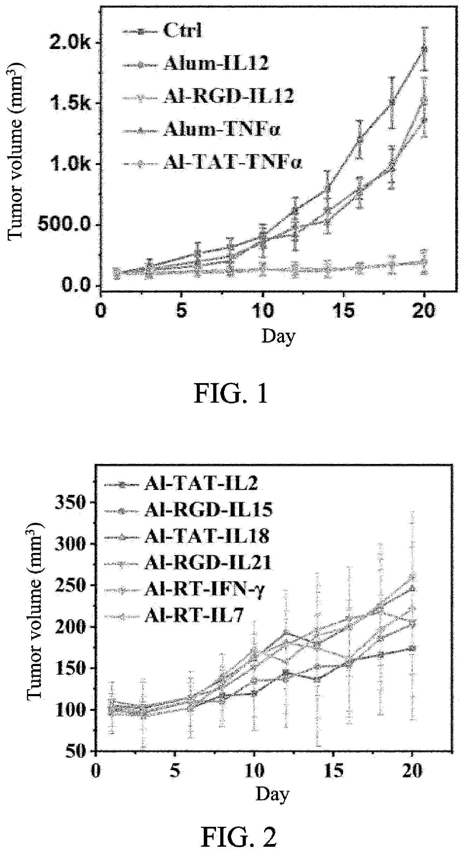

The results are shown in Fig. 1, which is an analysis of the effect of the aluminum nanocrystalline composite immune drug constructed on inhibiting tumor growth.

As shown in Fig. 1, on Day 20, the average tumor size of mice in the control group (Ctrl group) is 1942 mm³; the average tumor volume size of the Alum-II,12 group is 1364 mm³; while the average tumor volume size of the Al-RGD-IL12 group is 183 mm³; the average tumor volume size of the Alum-TNFα group is 1540 mm³; while the average tumor volume size of the Al-TAT-TNFα group is 204 mm³; it can be seen that the constructed cytokine-carrying aluminum nanocrystalline composite immune drug can effectively inhibit tumor growth.

### Example 16

Under the premise of following the national animal health agreement, BALB/c mice aged 6-8 weeks were selected for posterior subcutaneous tumor implantation. The tumor model was selected from B16F10 mouse melanoma highly metastatic cells. When the implanted tumor tissue grew to about 100 mm³, the mice were randomly divided into 5 groups and injected with 50 µL of the aluminum nanocrystalline composite immune drug containing 0.25 µg cytokine or the gamma interferon constructed in Examples 8-13 intratumorally. The changes in the tumor volume of the mice were recorded.

The results are shown in Fig. 2, which is an analysis of the effect of the aluminum nanocrystalline composite immune drug constructed in Examples 8-13 on inhibiting tumor growth.

As shown in Fig. 2, on Day 20, the average tumor volume size of mice treated with the aluminum nanocrystalline composite immune drugs constructed in Examples 8-13 is 181 mm³-254 mm³, which is significantly lower than the average tumor volume of the control group shown in Example 15; it can be seen that the aluminum nanocrystalline composite immune drugs constructed in Examples 8-13 can also effectively inhibit tumor growth.

Tumor inhibition ability is the most direct indicator for verifying delivery efficiency. The aluminum nanocrystalline composite immune drugs obtained in Examples 6 to 13 of the present invention can all significantly inhibit the growth of melanoma. Since their components are similar and their mechanisms of action are substantially similar, only the Al-RGD-IL12 composite immune drug constructed in Example 5 and the Al-TAT-TNFα composite immune drug constructed in Example 6 are selected for subsequent further mechanism verification.

### Example 17

For each group of mice in Example 15, the cumulation and accumulation of cytokines in their tumor tissues were analyzed. 24 hours and 48 hours after the intratumoral injection of the drug, one mouse was randomly taken out from each of the four groups ②, ③, ④, and (5) in Example 15, and the tumor tissues were obtained by dissection after humanitarian sacrifice. The stock levels of the interleukin 12 (IL-12) or the tumor necrosis factor (TNFα) in the tumor tissues were analyzed according to the instructions of the ELISA kit for the interleukin 12 (IL-12) or tumor necrosis factor (TNFα).

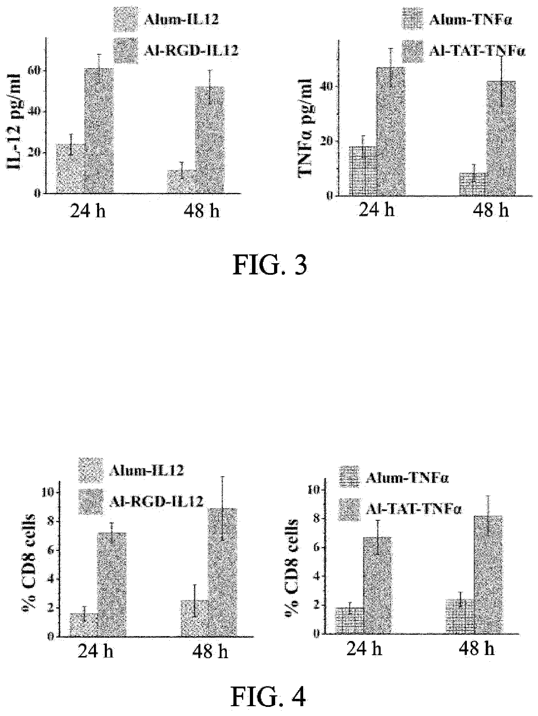

The results are shown in Fig. 3, which is an analysis of cytokine accumulation in the mouse tumor model in Example 15. As shown in Fig. 3, at 24 hours, the stock level of the interleukin 12 in the tumor tissue is 24.1 pg/mL for the Alum-IL12 experimental group; and 60.9 pg/mL for the Al-RGD-IL12 experimental group. At 48 hours, the stock level of the interleukin 12 in the tumor tissue is 11.4 pg/mL for the Alum-IL12 experimental group; and 52.3 pg/mL for the Al-RGD-IL12 experimental group. At 24 hours, the stock level of the tumor necrosis factor in the tumor tissue is 18.2 pg/mL for the Alum-TNFα experimental group; and 46.7 pg/mL for the Al-TAT-TNFα experimental group. At 48 hours, the stock level of the tumor necrosis factor in the tumor tissue is 8.4 pg/mL for the Alum-TNFα experimental group; and 41.5 pg/mL for the Al-TAT-TNFα experimental group. From the data, it can be seen that the aluminum nanocrystalline composite immune drug constructed can significantly improve the stability and accumulation time of cytokines in the tumor environment.

### Example 18

The proliferation of CD8+T cells was studied on the tumor tissues of mice after drug injection extracted in Example 15. A part of the tumor tissues was weighed and ground, then filtered through a 100 µm cell filter. The cell suspension was stained for surface markers with anti-CD8 and then analyzed on a flow cytometer. The results are shown in Fig. 4, which shows the proliferation of CD8 T cells in tumor tissues in the mouse tumor model in Example 15. As shown in Fig. 4, at 24 hours, the percentage level of CD8 T cells in the tumor tissue is 1.5% for the Alum-IL12 experimental group; 7.1% for the Al-RGD-IL12 experimental group; 1.7% for the Alum-TNFα experimental group; and 6.6% for the Al-TAT-TNFα experimental group. At 48 hours, the percentage level of CD8 T cells in the tumor tissue is 2.4% for the Alum-II,12 experimental group; 9.0% for the Al-RGD-IL12 experimental group; 2.3% for the Alum-TNFα experimental group; and 8.4% for the Al-TAT-TNFα experimental group. From the data, it can be seen that the aluminum nanocrystalline composite immune drug constructed can significantly improve the proliferation ability of CD8 T cells and effectively regulate the immune response of tumor tissues.

### Example 19

The Aluminum nanocrystalline composite immune drug inhibits distal tumor growth.

The B16F10 mouse melanoma cells were inoculated subcutaneously into the right hind leg of Balb/c mice, which were recorded as the in-situ tumor. Half of the B16F10 mouse melanoma cells were inoculated subcutaneously into the left hind leg of Balb/c mice, which were recorded as the distal tumor. When the average volume of the in-situ tumor grew to 100 mm³, the average volume size of the distal tumor was about 50 mm³, and then the mice were randomly divided into 5 groups and 50 µL of the following drugs were injected into the in-situ tumor: ①, saline for injection (Ctrl); ②, a simple mixture of the commercial aluminum adjuvant Alum and 0.25 µg IL-12 (Alum-IL12); ③, the Al-RGD-IL12 composite immune drug containing 0.25 µg IL-12 constructed in Example 6; ④, a simple mixture of commercial aluminum adjuvant Alum and 0.25 µg TNFα (Alum-TNFα); and (5), the Al-TAT-TNFα composite immune drug containing 0.25 µg IL-12 constructed in Example 7. The mice were treated every 3 days, and the changes in the volume of the distal tumor of the mice were recorded.

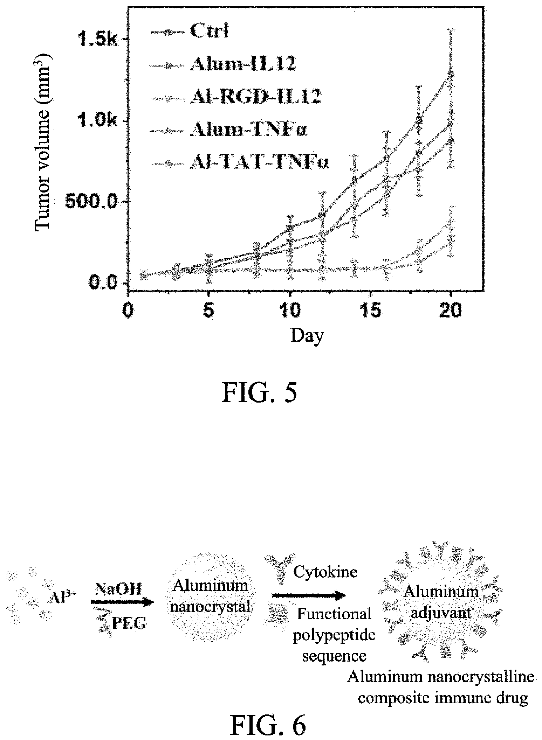

The results are shown in Fig. 5, which is an analysis of the effect of the aluminum nanocrystalline composite immune drug constructed on inhibiting distal tumor tissue growth. As shown in Fig. 5, on Day 20, the average size of the distal tumor of mice in the control group is 1286 mm³; the average volume size of the distal tumor of the Alum-IL12 group is 984 mm³; while the average volume size of the distal tumor of the Al-RGD-IL12 group is 253 mm³; the average volume size of the distal tumor of the Alum-TAT-TNFα group is 880 mm³; while the average volume size of the distal tumor of the Al-TAT-TNFα group is 382 mm³; it can be seen that the constructed cytokine-carrying aluminum nanocrystalline composite immune drug can effectively inhibit distal tumor growth. This indicates that the aluminum nanocrystalline composite immune drug constructed not only has a significant inhibitory effect on the growth of existing tumors, but also has a significant inhibitory effect on the growth of neoplasms.

The above is a detailed introduction to the aluminum nanocrystalline composite immune drug provided by the present invention and a preparation method therefor and use thereof. This article uses specific examples to illustrate the principles and embodiments of the present invention. The description of the above examples is only used to help understand the method of the present invention and its core concept. It should be noted that for those of ordinary skill in the art, several modifications and improvements can be made without departing from the concept of the present invention, which all fall within the protection scope of the present invention.

## Claims

1. An aluminum nanocrystalline composite immune drug, which is constructed by binding a functional polypeptide sequence and a cytokine molecule based on aluminum nanocrystals by means of polyethylene glycol molecule.

2. The aluminum nanocrystalline composite immune drug according to claim 1, wherein the functional polypeptide sequence comprises a polypeptide sequence which can specifically bind to a protein highly expressed on the surface of tumor cells.

3. The aluminum nanocrystalline composite immune drug according to claim 2, wherein the functional polypeptide sequence is one or both of a circular RGD sequence and a TAT sequence that can assist an immune drug to enter cells.

4. The aluminum nanocrystalline composite immune drug according to claim 1, wherein the cytokine molecule is selected from one or more of interleukin factors including IL-2, IL-7, IL-12, IL-15, IL-18, IL-21, gamma interferon IFN-γ, or tumor necrosis factor TNFα.

5. The aluminum nanocrystalline composite immune drug according to claim 1, wherein the aluminum nanocrystals are prepared by the following steps:
stirring and mixing an aluminum salt solution and a polyethylene glycol solution, and adding an aqueous NaOH solution to adjust the pH value, and then allowing them to stand to obtain a precipitate, which is then centrifuged, washed and purified to obtain polyethylene glycol-modified aluminum nanocrystals.

6. The aluminum nanocrystalline composite immune drug according to claim 5, wherein the solute of the aluminum salt solution is one or more of aluminum chloride, aluminum nitrate, aluminum sulfate, and aluminum acetate; the solvent used for the aluminum salt solution is pure water or a sodium acetate solution with a concentration of 0.01 mol/L.

7. The aluminum nanocrystalline composite immune drug according to claim 5, wherein the polyethylene glycol solution is a mixed reagent of polyethylene glycol modified with phosphoserine and polyethylene glycol with maleimide group and terminal carboxylic acid.

8. The aluminum nanocrystalline composite immune drug according to claim 5, wherein the aqueous NaOH solution is added to adjust the pH to 5.0-8.0.

9. A method for preparing the aluminum nanocrystalline composite immune drug according to any one of claims 1 to 8, comprising the following steps:
(1) adding aluminum nanocrystals and a functional polypeptide sequence into a solvent, stirring for the reaction, and centrifuging, washing and purifying, then resuspending into ultrapure water to obtain a functional polypeptide sequence-modified aluminum nanocrystalline solution; and
(2) adding a cytokine molecule into an MES buffer, and adding EDC/NHS to activate the carboxyl groups on the cytokine molecule, and then adding the aluminum nanocrystalline solution to form a covalent amide bond between the amino group of M-PEG-NH₂and the carboxyl group on the cytokine molecule, thereby obtaining a cytokine molecule-modified aluminum nanocrystalline composite immune drug.

10. The method for preparing the aluminum nanocrystalline composite immune drug according to claim 9, wherein the solvent is 0.1 M phosphate buffer or 0.1 M ammonium acetate buffer.

11. The method for preparing the aluminum nanocrystalline composite immune drug according to claim 9, wherein the functional polypeptide sequence comprises a polypeptide sequence which can specifically bind to a protein highly expressed on the surface of tumor cells.

12. The method for preparing the aluminum nanocrystalline composite immune drug according to claim 9, wherein the cytokine molecule is selected from one or more of interleukin factors including IL-2, IL-7, IL-12, IL-15, IL-18, IL-21, gamma interferon IFN-γ, or tumor necrosis factor TNFα.

13. The method for preparing the aluminum nanocrystalline composite immune drug according to claim 9, wherein the functional polypeptide sequence is one or both of a circular RGD sequence and a TAT sequence that can assist an immune drug to enter cells.

14. Use of the aluminum nanocrystalline composite immune drug according to any one of claims 1 to 8 or the aluminum nanocrystalline composite immune drug prepared by the preparation method according to any one of claims 9 to 13 in immunotherapy in tumor models.

15. The use according to claim 14, wherein the use is intravenous injection, mucosal administration, subcutaneous injection, intradermal injection, peritumoral injection, or intratumoral injection.

16. A method for immunotherapy of tumors, comprising administering the aluminum nanocrystalline composite immune drug according to any one of claims 1 to 8 or the aluminum nanocrystalline composite immune drug prepared by the preparation method according to any one of claims 9 to 13.

17. The method according to claim 15, wherein the mode of the administration comprises: intravenous injection, mucosal administration, subcutaneous injection, intradermal injection, peritumoral injection, or intratumoral injection.
